# EUROPÄISCHE PATENTSCHRIFT

(11) **EP 1 032 799 B1**
(45) Veröffentlichungstag und Bekanntmachung des Hinweises auf die Patenterteilung: **12.05.2004**
(21) Anmeldenummer: 98966152.5
(22) Anmeldetag: 18.11.1998
(51) Int. Cl.: F26B 5/06

(54) **VERFAHREN ZUR HERSTELLUNG PORÖSER STRUKTUREN**
METHOD FOR PRODUCING POROUS STRUCTURES
PROCEDE DE PRODUCTION DE STRUCTURES POREUSES

(30) Priorität: 19.11.1997 DE 19751031
(43) Veröffentlichungstag der Anmeldung: 06.09.2000
(73) Patentinhaber: Heschel, Ingo, 52134 Herzogenrath (DE); Rau, Günter, Prof. Dr., D-52066 Aachen (DE)
(72) Erfinder: Heschel, Ingo, 52134 Herzogenrath (DE); Rau, Günter, Prof. Dr., D-52066 Aachen (DE)
(74) Vertreter: Castell, Klaus, Dr.-Ing.
(86) Internationale Anmeldenummer: PCT/DE1998/003403
(87) Internationale Veröffentlichungsnummer: WO 1999/027315

(56) Entgegenhaltungen:
- EP-A- 0 317 411
- DE-A- 2 625 289
- DE-A- 4 028 622
- DE-A- 4 328 329
- US-A- 2 610 625
- US-A- 3 157 524
- US-A- 3 300 994
- US-A- 3 477 137
- US-A- 3 864 840
- US-A- 4 501 719
- US-A- 4 531 373
- US-A- 4 590 684
- PATENT ABSTRACTS OF JAPAN vol. 007, no. 167 (C-177), 22. Juli 1983 & JP 58 074103 A (YAMANOUCHI SEIYAKU KK), 4. Mai 1983

## Beschreibung

Die Erfindung betrifft ein Verfahren zur Herstellung von Schwämmen bei dem eine flüssige oder pastöse Mischung von Substanzen zumindest partiell zur Erstarrung gebracht wird und anschließend gefriergetrocknet wird.

Zur Herstellung von Schwämmen für kosmetische und medizinische Zwecke ist es bekannt, eine wäßrige Ausgangssuspension durch Temperaturabsenkung einzufrieren und anschließend gefrierzutrocknen. Dabei wird wie Ausgangssuspension in Metallschalen gefüllt, die anschließend von unten durch eine kalte Sole abgekühlt werden, so daß die Ausgangssuspension erstarrt. Die Suspension erstarrt dabei zunächst am Boden der Schale und danach auch in der gesamten Schale. Dabei entsteht durch das Eiswachstum eine Erstarrungsstruktur, die nach der Gefriertrocknung zu einem sehr unregelmäßigen porösen Schwamm mit unterschiedlicher Dichteverteilung und unterschiedlicher Porenausrichtung führt.

Es ist auch bekannt, in Fläschchen Produkte gefrierzutrocknen. Dabei entsteht innerhalb der Fläschchen in radialer Richtung eine sehr unterschiedliche Erstarrungsstruktur, da im äußeren Bereich der Fläschchen eine Unterkühlung eintritt, die zu globulitischen Eiskristallen führt, während im inneren Bereich der Fläschchen die Erstarrung dendritisch oder zellular erfolgt.

Diese unterschiedlichen Porositäten innerhalb des Produktes behindern die Gefriertrocknung, da weniger poröse Strukturbereiche starke Stofftransportwiderstände erzeugen. Außerdem sind für viele technische und medizinische Anwendungen Produkte mit homogener Porosität von großer Bedeutung.

Der Erfindung liegt daher die Aufgabe zugrunde, das eingangs beschriebene Verfahren so weiterzuentwickeln, daß Produkte mit einer homogeneren Struktur erzeugt werden können.

Diese Aufgabe wird mit einem gattungsgemäßen Verfahren gelöst, bei dem die Mischung zwischen zwei temperierbaren, beabstandet voneinander angeordneten Flächen unterschiedlicher Temperaturen abgekühlt wird, dadurch gekennzeichnet, daß eine genaue Temperaturregelung durchgeführt wird, derart, daß während des Erstarrungsprozesses eine geordnete Struktur ersteht.

Diese spezielle Temperaturführung bewirkt ein Kristallstrukturwachstum von der kälteren zur wärmeren Fläche. Dabei wächst die Kristallfront kontinuierlich von der einen zur anderen Fläche und läßt in der Probe eine homogene Verteilung zwischen kristallinen und nicht kristallinen Strukturen entstehen. Die anschließende Gefriertrocknung wird durch die homogene Kristallstruktur erleichtert und legt durch Verdampfung bzw. Sublimation der kristallinen und flüssigen Komponenten die homogene poröse Struktur frei. Durch die Einstellung des Temperaturgradienten bzw. der Erstarrungsfrontgeschwindigkeit ist die entstehende Struktur optimal beeinflußbar.

Vorteilhaft ist es, wenn die Differenz der Temperaturen sich gegenüberliegender Flächenbereiche während der Abkühlung im wesentlichen konstant gehalten wird. Eine konstante Temperaturdifferenz hat eine nahezu konstante Geschwindigkeit der Fortbewegung der Eisfront zwischen den Flächen zur Folge und führt somit zu einer besonders homogenen porösen Struktur.

Gerade bei biologischem Probematerial ist es häufig nicht möglich, die Temperatur auf über 37 - 40° C zu erhöhen, da sonst Zellschädigungen oder Denaturierungen auftreten. In diesem Fall ist es sinnvoll, wenn die Temperatur eines Flächenbereiches solange konstant auf einer maximal zulässigen, unschädlichen Temperatur gehalten wird, bis der andere Flächenbereich soweit abgekühlt ist, daß der gewünschte Gradient aufgebaut ist. Dadurch wird eine zell- bzw. proteinschädigende Temperatur in der Mischung vermieden.

Eine einfache Art der Durchführung des Verfahrens sieht vor, daß die Flächen parallel zueinander angeordnet sind. Zwischen diese parallel angeordneten Flächen wird die Mischung gebracht und so abgekühlt, daß die Kristallbildung von einer Fläche zur anderen fortschreitet.

Um eine weitere Vereinfachung der Verfahrensdurchführung zu erhalten, kann die wärmere Flächenbegrenzung der Mischung auch direkt durch Wärmestrahlung oder Konvektion temperiert werden.

Je nach benötigter poröser Struktur kann es jedoch auch von Vorteil sein, wenn die Flächen konzentrisch zueinander angeordnet sind. Dabei kann entweder die innere oder äußere Fläche wärmer gehalten werden, so daß die Kristallbildung entweder von innen nach außen oder von außen nach innen fortschreitet.

Je nach Anordnung der Flächen entstehen radial verlaufende oder parallel zueinander verlaufende Kristallstrukturen, die sich über den gesamten Bereich zwischen den Flächen erstrecken können oder nur einen Teil des Bereiches ausfüllen.

Eine besonders vorteilhafte Ausgestaltung der Erfindung sieht vor, daß die Mischung auf einer ersten Seite zwischen den den Flächen eingeleitet wird und auf einer zweiten Seite zwischen Flächen herausgelassen wird, wobei von der ersten Seite zur zweiten Seite die Temperatur der sich gegenüberliegenden Flächenbereiche abfällt. Dies ermöglicht sowohl bei parallel zueinander angeordneten als auch konzentrisch zueinander angeordneten Flächen eine kontinuierliche Erstarrung einer flüssigen oder pastösen Mischung. Im Idealfall wird an einer Stelle der Vorrichtung die flüssige oder pastöse Mischung eingefüllt und am anderen Ende kontinuierlich die poröse Struktur aus der Vorrichtung entnommen.

Insbesondere bei kontinuierlichen Verfahren ist es vorteilhaft, wenn die Mischung durch wärmeleitende Transportbänder oder Schläuche geleitet oder mit ihnen geführt wird. An den sich gegenüberliegenden Flächen geführte Transportbänder oder Schläuche erlauben einen kontinuierlichen oder diskontinuierlichen Transport der Mischung relativ zu den Flächen und verhindern einen direkten Kontakt zwischen den Flächen und der Mischung. Dabei kann die Probe entweder relativ zu den Bändern oder Schläuchen bewegt werden oder zusammen mit den Bändern oder Schläuchen an den Flächen vorbeigeführt werden.

Eine besonders einfache Art der Gefriertrockung wird dadurch ermöglicht, daß das zumindest partiell erstarrte Produkt gefriergetrocknet wird, indem eine Fläche entfernt wird und die andere Fläche temperiert wird. Nach Entfernung einer Fläche ist das zumindest partiell erstarrte Produkt frei zugänglich für einen angelegten Unterdruck, der durch Sublimations-, Desorptions- und Diffusionsvoränge die Eisphase und weiteres ungefrorenes Wasser aus der aufkonzentrierten Phase entfernt.

Die schon zur Einstellung des Temperaturgradienten während des Erstarrungsprozesses temperierte Fläche wird auch während der Gefriertrocknung temperiert, um den Gefriertrocknungsvorgang zu steuern.

Die Erfindung sieht vor allem vor, daß als flüssige oder pastöse Mischung eine wäßrige Lösung oder Suspension verwendet wird. Die wäßrige Phase ist leicht durch Gefriertrocknung zu entfernen und erlaubt die Herstellung von vor allem in der Medizintechnik vielfältig einsetzbaren porösen Strukturen, wie bspw. Schwämmen.

Werden der flüssigen oder pastösen Mischung keine Zusatzstoffe zur Beeinflussung der Kristallstruktur zugegeben, kann der Prozeß so gestaltet sein, daß es nicht zu einer Umorientierung oder Verschiebung der suspendierten Fremdphasenteilchen wie bspw. Fasern durch das Kristallwachstum kommt. Somit können durch das erfindungsgemäße Verfahren sehr geordnete Strukturen erzeugt werden, mit einer sehr homogenen Verteilung der Fremdphasenteilchen, die im wesentlichen der Verteilung in der flüssigen oder pastösen Mischung entspricht. Nach der Gefriertrockung erhält man so sehr feine Strukturen, die bspw. als Schwämme bei der Blutstillung oder Verbrennungswundbehandlung aber auch als Biomaterial für die Knorpelzüchtung vorteilhaft sein können.

Für andere Anwendungen ist es besonders vorteilhaft, wenn der flüssigen oder pastösen Mischung Wirkstoffe oder Zusatzstoffe zugegeben werden. So können bspw. Salze oder Säuren zur Beeinflussung der Kristallstruktur der flüssigen Mischung zugegeben werden. Bei dem erfindungsgemäßen Verfahren wachsen dann z. B. fingerförmige oder bäumchenartige (dendritische) Eiskristalle durch die flüssige oder pastöse Mischung, die eine Umorientierung der suspendierten Fremdphasenteilchen bewirken können. Bei der anschließenden Gefriertrocknung sublimieren diese fingerförmigen Eiskristalle und es verbleiben homogene offene Poren. Die Eiskristallstruktur und damit die spätere Porenstruktur kann durch die Prozeßführung bei der Abkühlung (Temperaturgradient über der Probe und Eisfrontgeschwindigkeit) sowie die Art und Konzentration der Zusatzstoffe beeinflußt werden.

Zur Beeinflussung der Festigkeitseigenschaften der erzeugten Strukturen können weitere Zusatzstoffe ergänzt werden. Im Falle von beispielsweise Kollagensuspensionen lassen sich durch Zugabe von bspw. Hydroxyapatit härtere Strukturen erzeugen, die die Eignung des Schwammes als Knochenersatzmaterial verbessern. Ferner ist es z. B. bei medizinischen Implantaten vorteilhaft, schon der flüssigen Mischung biologisch aktive Stoffe oder gar lebende Zellen mit Gefrierschutzadditiven hinzuzugeben, die beim Einsatz der porösen Strukturen helfen, die gewünschten biologischen Prozesse zu verbessern. Um eine Infektabwehr zu erzielen, können dabei z. B. Antibiotika zugegeben werden. Um die Wundheilung z. B. durch Besiedelung der Strukturen mit Zellen oder das Hineinwachsen von Gefäßen oder Nerven zu beschleunigen, können als biologisch aktive Stoffe Pepide, Proteine oder Enzyme zugegeben werden, die z. B. die Zelladhäsion oder das Zellwachstum in den Strukturen fördern. Biologische Zellen wie bspw. Chondrozyten, Fibroblasten, Keratinozyten und Endothelzellen fördern die Bildung einer neuen Zellmatrix bzw. eines Zellmonolayers oder Gefäßes und produzieren für die Wundheilung wichtige Stoffe. Hydroxyethylstärke als Beispiel für ein makromolekulares Kyroprotektiv - eventuell ergänzt durch Zugabe bestimmter Zucker - schränkt die Zellschädigung beim Gefrierprozeß ein und ist ferner zur Gefriertrocknung geeignet. Die so erzeugten Schwämme können nach Rehydrierung durch Körperflüssigkeiten oder Lösungen spezielle biologische oder medizinische Funktionen erfüllen.

Die oben erwähnten Wirkstoffe oder Zusatzstoffe, biologisch aktiven Materialien oder lebenden Zellen können auch der erzeugten, gefriergetrockneten Struktur zugegeben werden, da der poröse Aufbau eine Einlagerung dieser Substanzen in die Struktur erleichtert.

Eine besonders vorteilhafte Verwendung des erfindungsgemäßen Verfahrens liegt im Einsatz für medizinische oder pharmazeutische Produkte oder Produkte der Umwelt- und Biotechnologie. Hierbei sind Schwämme für kosmentische oder medizinische Zwecke insbesondere im Bereich des Tissue Engineering von besonderer Bedeutung.

Eine weitere Möglichkeit zur Lösung der erfindungsgemäßen Aufgabe liegt darin, poröse Strukturen mit einem Verfahren herzustellen, bei dem eine flüssige oder pastöse Mischung von Substanzen zumindest partiell zur Erstarrung gebracht wird und anschließend gefriergetrocknet wird, wobei die Mischung unter Überdruck vorgekühlt wird und anschließend schlagartig entspannt wird.

Die Mischung wird dabei vorteilhafterweise bis kurz vor den Erstarrungspunkt bei erhöhtem Druck gekühlt und die schlagartige Entspannung führt zu einer Kristallisation der unterkühlten flüssigen Mischung, wobei sich im gesamten Bereich der Mischung gleichzeitig homogen verteilt Eiskristalle bilden, die je nach Temperatur und Druckführung weiter wachsen oder klein bleiben und durch die Gefriertrocknung entfernt werden, so daß eine homogene poröse Struktur entsteht. Die Temperatur und Druckführung erlaubt es, entweder eine Vielzahl kleinerer Kristalle oder wenige größere Kristalle zu erzeugen, so daß feinporige oder grobporige Strukturen entstehen. Vorteilhaft ist es, wenn der Überdruck im Bereich zwischen 1 und 250 MPa liegt.

Werden zusätzliche Gase in der flüssigen Mischung gelöst, so kann die Kristallisation genutzt werden, um durch Gasblasennukleation eine größere Porosität und geringere Dichte der Struktur zu erhalten. Diese Möglichkeit zur Erzeugung größerer Porosität besteht sowohl bei der Variante mit Druckabsenkung als auch bei der zuvor beschriebenen Variante ohne Druckabsenkung.

Eine weitere vorteilhafte Variante zur Erzeugung größerer Porosität ist es, nach der erfindungsgemäßen Erstarrung eine Rekristallisation der Probe in der Nähe der Erstarrungstemperatur vorzunehmen, und erst dann den Gefriertrocknungsschritt anzuschließen. Hierdurch können kleinere kristalline Bereiche zu größeren zusammenwachsen, wodurch größere Poren entstehen.

Für alle Varianten ergibt sich zudem die Möglichkeit, Fremdmaterialien in gewünschter Geometrie wie bspw. Fäden in die flüssige oder pastöse Mischung einzubringen, die nach der Gefriertrocknung aus der Probe entfernt werden können, so daß die poröse Struktur zusätzlich durch die Geometrie und Lage der Fremdmaterialien bestimmt wird.

Ein Ausführungsbeispiel des erfindungsgemäßen Verfahrens ist in der Zeichnung dargestellt und wird im folgenden näher erläutert.

Es zeigt
- Figur 1: eine Vorrichtung für die Herstellung poröser Strukturen im Batch-Prozeß,
- Figur 2: ein Beispiel für die Temperaturführung an verschiedenen Stellen der Proben über der Zeit,
- Figur 3: eine Vorrichtung für die Herstellung poröser Strukturen im kontinuierlichen Prozeß mit darüber schematisch aufgetragener Temperaturführung,
- Figur 4: eine weitere Vorrichtung für die Herstellung poröser Strukturen im kontinuierlichen Prozeß,
- Figur 5: eine homogene gleichmäßige Struktur einer wäßrigen Kollagensuspension,
- Figur 6: eine homogene fingerförmige Eiskristallstruktur einer wäßrigen Kollagensuspension und
- Figur 7: eine Darstellung der Porengröße über der Essigsäurekonzentration.

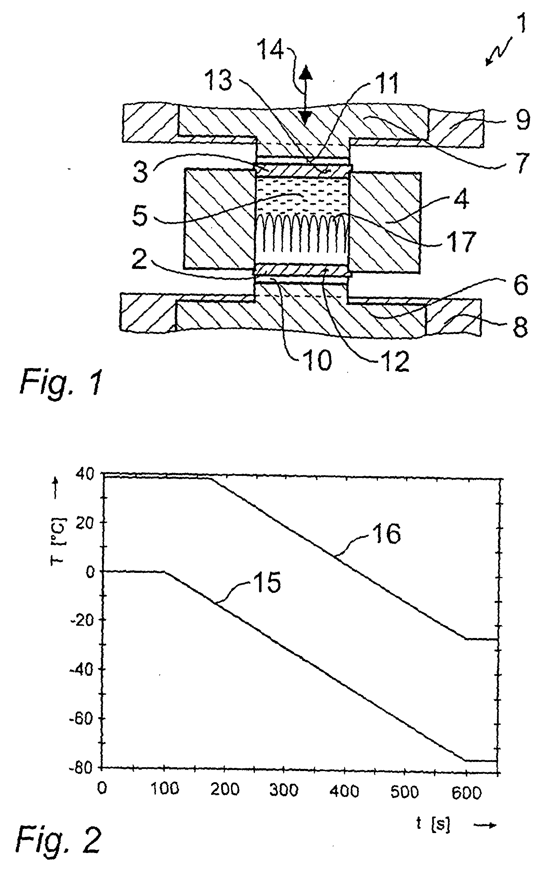

Die in Figur 1 gezeigte Vorrichtung 1 hat eine untere Probenbegrenzung 2 und eine obere Probenbegrenzung 3, zwischen denen innerhalb eines isolierenden Probenrahmens 4 der Probenraum angeordnet ist: Die oberen und unteren Probenbegrenzungen 2 bzw. 3 sind identisch aufgebaut und dienen der Temperierung der zwischen diesen Probenbegrenzungen im Probenraum 5 angeordneten Probe während des Erstarrungsvorgangs und bei Bedarf auch während des Gefriertrocknungsvorgangs. Die Temperierung erfolgt über Kupferblöcke 6 bzw. 7, die seitlich in eine Wärmeisolationsummantelung 8 bzw. 9 eingebettet sind. Zwischen den Kupferblöcken 6 bzw. 7 und der unteren bzw. oberen Probenbegrenzung 2 bzw. 3 befindet sich jeweils ein elektrisches Folienheizelement 10 bzw. 11 zur Kompensationsheizung. Die Probenbegrenzungen 2 bzw. 3 besitzen Temperaturmeßstellen 12 bzw. 13, so daß mittels der gekühlten Kupferblöcke 6 bzw. 7 und den Folienheizelementen 10 bzw. 11 eine geneau Temperaturregelung durchgeführt werden kann.

Zum Einfüllen und Entnehmen der Probe aus dem Probenraum 5 kann die obere Probenbegrenzung 3 mittels einer Spindel 14, die in der Zeichnung nur schematisch als Doppelpfeil dargestellt ist, pneumatisch oder hydraulisch angehoben werden.

Die Vorrichtung kann in einer geschlossenen Vakuumkammer mit Kondensator (nicht dargestellt) aufgestellt werden, so daß bei Bedarf der Gefriertrocknungsprozeß direkt im Anschluß durchgeführt werden kann. Bei der Gefriertrocknung wird die Vakuumkammer evakuiert und zur Probentemperierung während der Gefriertrocknung kann bspw. das untere Folienheizelement 10 verwendet werden.

In Figur 2 sind die Temperaturverläufe schematisch über der Zeit aufgetragen. Die untere Kurve 15 zeigt den Temperaturverlauf an der unteren Probenbegrenzung 2 und die obere Kurve 16 zeigt den Temperaturverlauf an der oberen Probenbegrenzung 3. Da die Probenbegrenzungen in einem Abstand von 10 mm voneinander entfernt angeordnet sind, wird die untere Kurve 15 auch mit "x = 0 mm" und die obere Kurve 16 mit "x = 10 mm" beschrieben.

Mittels der Kupferblöcke 6 bzw. 7 und der Heizelemente 10 bzw. 11 werden die Probenbegrenzungen 2 bzw. 3 in Abhängigkeit von der Zeit genau auf den im Diagramm dargestellten Temperaturen gehalten. Dabei wächst die Eisfront 17 im dargestellten Fall mit einer zellularen Struktur von der unteren zur oberen Probenbegrenzung. Die Temperatur wird dabei so eingestellt, daß die Differenz zwischen der Temperatur an der unteren Probenbegrenzung 2 und der Temperatur an der oberen Probenbegrenzung 3 konstant gehalten wird. Da jedoch eine bestimmte Maximaltemperatur zur Vermeidung von Zellschädigungen nicht überschritten werden darf, ist im Anfangszeitraum der Abkühlung die Temperaturdifferenz etwas kleiner gehalten. Dadurch wird eine geregelte Temperaturabsenkung mit konstanter Kühlrate eingestellt. Dadurch wird erzielt, daß die Eisfront mit nahezu konstanter Geschwindigkeit durch die Probe wächst, so daß in der gesamten Probe sehr regelmäßige Eisstrukturen entstehen, deren Geometrie sich unter anderem durch die Wahl des Temperaturgradienten zwischen der unteren und der oberen Begrenzungsfläche und durch die Kühlrate, mit der die Temperaturen der beiden Begrenzungen abgesenkt werden, beeinflussen läßt.

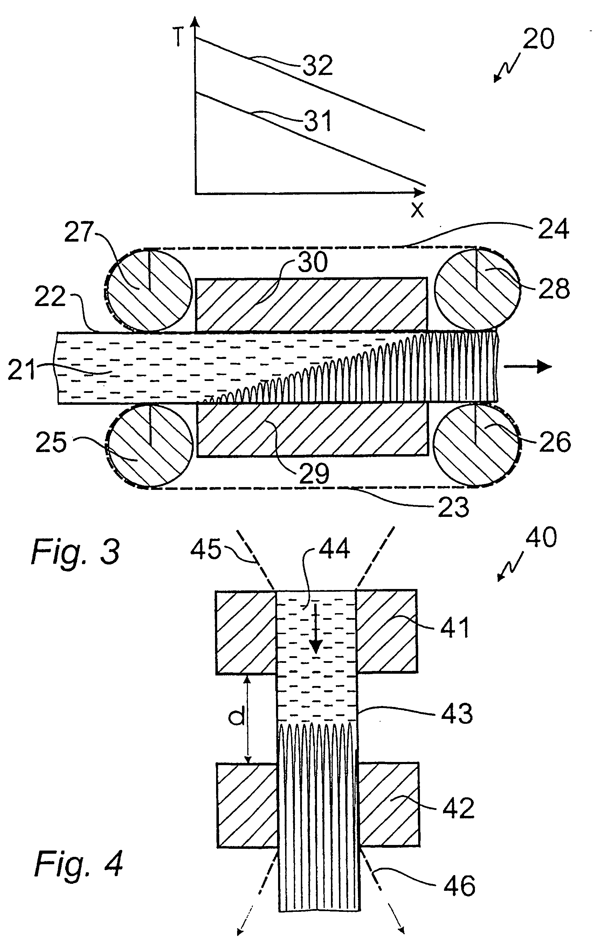

Die Figur 3 zeigt eine kontinuierliche Vorrichtung 20. Als Probenraum 21 dient in diesem Fall ein Schlauch 22, der zwischen zwei Transportbändern 23, 24 geführt ist. Die Transportbänder 23 und 24 werden jeweils von zwei Antriebswalzen 25, 26 bzw. 27, 28 angetrieben. Zwischen diesen Walzen ist an der Oberseite und an der Unterseite des Schlauches 22 jeweils ein Kühlelement 29 bzw. 30 angeordnet. Diese Kühlelemente 29 bzw. 30 sind so aufgebaut, daß sie in ihrer Längserstreckung einen Temperaturgradienten erzeugen, der auf den Probenraum 21 wirkt. Die Kühlelemente 29, 30 können hierzu segmentiert aufgebaut sein, um eine stufenweise Temperaturabsenkung zu erzeugen. Vorteilhaft ist jedoch eine nahezu kontinuierliche Temperaturabsenkung, die in der über der Vorrichtung angedeuteten Grafik dargestellt ist. In dieser Grafik ist die Temperatur über der Längserstreckung der Kühlelemente 29, 30 aufgetragen. Die Gerade 31 zeigt den Temperaturverlauf an der Unterseite des Probenraumes 21 und die Gerade 32 zeigt den Temperaturverlauf an der Oberseite des Probenraumes 21. Diese Grafik zeigt, daß bei konstanten Abkühlraten die Temperaturdifferenz an sich gegenüberliegenden Orten zwischen den Kühlelementen 29, 30 konstant gehalten wird.

Bei der Verwendung dieser Vorrichtung 20 wird in den Probenraum 21 auf der linken Seite eine flüssige oder pastöse Mischung in den Schlauch 22 eingefüllt. Dieser Schlauch wird mittels der Förderbänder 23 und 24 mit kontinuierlicher Geschwindigkeit zwischen den Kühlelementen 29 und 30 hindurchgeführt, so daß durch die Abkühlung innerhalb des Schlauches 22 von der Unterseite zur Oberseite des Schlauches eine Eisfront wächst, die am Ende der Kühlelemente 29 und 30 die gesamte Probe durchwachsen haben sollte, so daß die gefrorene Probe anschließend in einer Vakuumkammer (nicht dargestellt) gefriergetrocknet werden kann.

Der Schlauch 22 kann eine abgerundete, rechteckige oder anderweitige Form aufweisen, so daß je nach Anwendungsfall spezielle Formteile, wie bspw. Stäbe oder Platten mit der Vorrichtung im kontinuierlichen Prozeß erzeugt werden können.

Die Vorrichtung 20 kann auch seitliche Begrenzungen aufweisen, so daß die die Probe ohne Schlauch zwischen den Kühlelementen führbar ist. Darüber hinaus kann die Probe auch zwischen zwei einzelnen Folien geführt werden.

Eine weitere kontinuierliche Vorrichtung 40 zeigt die Figur 4. Bei dieser Vorrichtung wird die Flüssigkeit zwischen zwei beabstandet angeordneten Kühlelementen 41, 42 in einem Rohr 43 geführt. Der Abstand D der Kühlelemente 41 und 42 führt zu einem Raum zwischen den Kühlelementen, in dem ein Temperaturgradient einstellbar ist. Bei Einfüllen eines flüssigen oder pastösen Mediums 44 an der trichterförmigen Oberseite 45 des Rohres 43 gelangt die Probe in den Raum zwischen den Kühlelementen 41 und 42, so daß beim Durchströmen des Rohres 43 sich zwischen den Kühlelementen eine Eisfront ausbildet. Dies führt dazu, daß an der trichterförmigen Unterseite 46 der Vorrichtung 40 die gefrorene Probe die Vorrichtung verläßt, um anschließend gefriergetrocknet zu werden.

Ähnlich wie bei der zuvor beschriebenen Vorrichtung 20 kann auch bei der seitlichen Anordnung gemäß der Vorrichtung 40 die Probe in einem Schlauch oder zwischen Folien geführt werden.

Auch bei den kontinuierlichen Vorrichtungen kann zur einfacheren und genaueren Regelung der Temperaturführung zwischen den Kühlelementen 29 bzw. 31 und 41 bzw. 42 und der Probe ein Folienheizelement angeordnet werden.

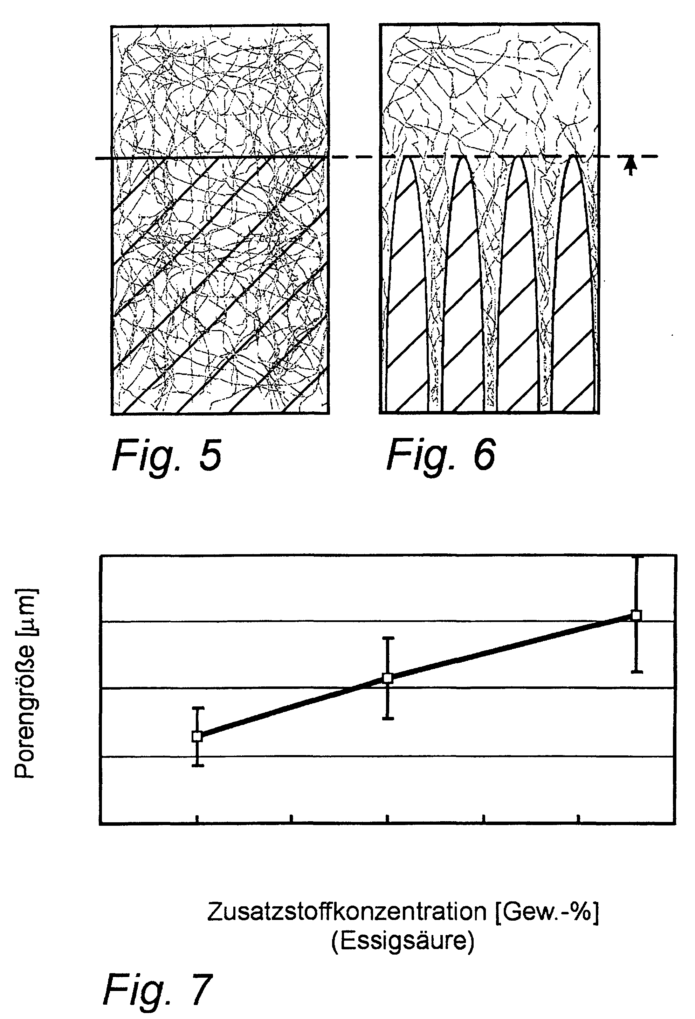

Die Figuren 5 und 6 zeigen zwei typische Varianten der bei der erfindungsgemäßen Abkühlung möglichen homogenen Strukturen am Beispiel einer schematisch dargestellten, wäßrigen Kollagensuspension. Im unteren Bereich der Figuren ist die kältere Seite und im oberen Bereich die wärmere Seite. Die Wachstumsrichtung der Erstarrungsfront geht somit von unten nach oben.

Vor Beginn der Erstarrung sind die Kollagenfasern relativ gleichmäßig verteilt. Werden der ca. 2gewichtsprozentigen Kollagensuspension keine Zusatzstoffe zugegeben, so läßt sich durch eine Prozeßtemperaturführung wie in Figur 2 beschrieben, ein ebenes Eisfrontwachstum erreichen, wobei die Faserlage und -verteilung, wie in Figur 5 dargestellt, im wesentlichen unverändert bleibt. In den Figuren ist der bereits kristallisierte Bereich schraffiert gekennzeichnet.

Werden der Kollagensuspension gefrierpunktserniedrigende Zusatzstoffe wie z. B. Essigsäuren oder Ethanol zugegeben, so kommt es bei richtiger Wahl der Erstarrungsparameter zur Bildung der in Figur 6 gezeigten fingerförmigen Eiskristallstrukturen, die ebenfalls schraffiert gekennzeichnet sind. Die Kollagenfasern verändern dabei ihre Orientierung und vernetzen sich zudem in den Kanälen zwischen den Eisfingern. Bei der Gefriertrocknung wird das Eis durch Sublimation entfernt und es verbleiben die gewünschten porösen Strukturen.

Durch die Art und Konzentratuion des Zusatzstoffes, durch die Konzentration des Kollagens, durch die Einstellung des Temperaturgradienten über der Mischung und durch die Wahl der Abkühlrate beider Probenbegrenzungen läßt sich die Eiskristallmorphologie und damit die Porengröße einstellen.

Die Figur 7 zeigt am Beispiel einer Suspension mit 1,8 Gewichtsprozent Kollagen, die bei einer Kühlrate von 9 K/min und einem Temperaturgradienten von 50 K/cm eingefroren wurde, den Einfluß des Zusatzstoffes Essigsäure auf die Porengröße. (Dabei ist als Porengröße stets der kürzeste Durchmesser der nicht kreisrunden Poren angegeben.) Auf der Abszisse ist die Zusatzstoffkonzentration in Gew-% (Essigsäure) und auf der Ordinate ist die Porengröße in µm angegeben.

### Optimale Parameterbereiche:

| | |
|---|---|
| Erzeugter Temperaturgradient | 1 K/cm bis 100 K/cm |
| vorteilhaft | 10 K/cm bis 60 K/cm |
| | |
| Zusatzstoffkonzentration | 0,1 Gew.-% bis 25 Gew.-% |
| vorteilhaft | 1 Gew.-% bis 10 Gew.-% |
| | |
| Abkühlrate | 0,1 K/min bis 500 K/min |
| vorteilhaft | 1 K/min bis 50 K/min |

## Patentansprüche

1. Verfahren zur Herstellung von Schwämmen, bei dem eine flüssige oder pastöse Mischung von Substanzen zumindest partiell zur Erstarrung gebracht wird und anschließend gefriergetrocknet wird, wobei die Mischung zwischen zwei temperierbaren, beabstandet voneinander angeordneten Flächen unterschiedlicher Temperaturen abgekühlt wird, ***dadurch gekennzeichnet, daß*** eine genaue Temperaturregelung durchgeführt wird, derart, daß während des Erstarrungsprozesses eine geordnete Struktur entsteht.

2. Verfahren nach Anspruch 1, ***dadurch gekennzeichnet, daß*** die Differenz der Temperaturen sich gegenüberliegender Flächenbereiche während der Abkühlung im wesentlichen konstant gehalten wird.

3. Verfahren nach Anspruch 1, ***dadurch gekennzeichnet, daß*** die Temperatur eines Flächenbereiches solange konstant gehalten wird, bis der andere Flächenbereich soweit abgekühlt ist, dass der gewünschte Gradient aufgebaut ist.

4. Verfahren nach einem der vorhergehenden Ansprüche, ***dadurch gekennzeichnet, daß*** die Flächen parallel zueinander angeordnet sind.

5. Verfahren nach einem der Ansprüche 1 bis 3, ***dadurch gekennzeichnet, daß*** die Flächen konzentrisch zueinander angeordnet sind.

6. Verfahren nach einem der vorhergehenden Ansprüche, ***dadurch gekennzeichnet, daß*** die Mischung auf einer ersten Seite zwischen den Flächen eingeleitet wird und auf einer zweiten Seite zwischen Flächen herausgelassen wird, wobei von der ersten Seite zur zweiten Seite die Temperatur der sich gegenüberliegenden Flächenbereiche abfällt.

7. Verfahren nach einem der vorhergehenden Ansprüche, ***dadurch gekennzeichnet, daß*** die Mischung durch wärmeleitende Transportbänder oder Schläuche geleitet oder mit ihnen geführt wird.

8. Verfahren nach einem der vorhergehenden Ansprüche, ***dadurch gekennzeichnet, daß*** das zumindest partiell erstarrte Produkt gefriergetrocknet wird, in dem eine Fläche entfernt wird und die andere Fläche temperiert wird.

9. Verfahren nach einem der vorhergehenden Ansprüche, ***dadurch gekennzeichnet, da*ß** als flüssige oder pastöse Mischung eine wäßrige Lösung oder Suspension verwendet wird.

10. Verfahren nach einem der vorhergehenden Ansprüche, ***dadurch gekennzeichnet, daß*** der flüssigen oder pastösen Mischung ein Wirkstoff oder Zusatzstoff zugegeben werden.

11. Verfahren nach einem der vorhergehenden Ansprüche, ***dadurch gekennzeichnet, daß*** der flüssigen oder pastösen Mischung biologisch aktive Materialien, lebende Zellen und/oder Kryoprotektive zugesetzt werden.

12. Verfahren nach einem der vorhergehenden Ansprüche, ***dadurch gekennzeichnet, daß*** der gefriergetrockneten Struktur ein Wirkstoff, biologisch aktive Materialien oder lebende Zellen zugegeben werden.

13. Verfahren nach einem der vorhergehenden Ansprüche, ***dudurch gekennzeichnet, daß*** zusätzlich Gase in der Mischung gelöst werden.

14. Verfahren nach einem der vorhergehenden Ansprüche, ***dadurch gekennzeichnet, daß*** die Mischung nach Abschluß der Abkühlung auf eine Rekristallisationstemperatur wiedererwärmt und für eine bestimmte Zeit gehalten wird, bevor sie gefriergetrocknet wird.

15. Verfahren nach einem der vorhergehenden Ansprüche, ***dadurch gekennzeichnet, daß*** Fremdmaterialien in gewünschter Geometrie und Orientierung in die flüssige oder pastöse Mischung eingebracht werden, die erst nach der Gefriertrocknung entfernt werden.

16. Verwendung des Verfahrens nach einem der Ansprüche 1 bis 15 zur Herstellung von medizinischen, pharmazeutischen Produkten oder Produkten der Umwelt- und Biotechnologie.

17. Verwendung des Verfahrens nach einem der Ansprüche 1 bis 15 zur Herstellung von Schwämmen für kosmetische oder medizinische Zwecke.

## Claims

1. A method of manufacturing sponges by which a fluid or pasty mixture of substances is at least partially solidified prior to being freeze-dried, said mixture being cooled down between two temperature regulatable spaced apart surfaces having different temperatures,
**characterized in that**
accurate temperature control is performed in such a manner that an ordered structure is obtained during the solidification process.

2. The method according to claim 1,
**characterized in that**
the difference in temperature between opposing surface areas is substantially kept constant during the cooling period.

3. The method according to claim 1,
**characterized in that**
the temperature of one surface area is kept constant until the other surface area has cooled down so as to obtain the desired gradient.

4. The method according to one of the afore mentioned claims,
**characterized in that**
the surfaces are disposed parallel to each other.

5. The method according to one of the claims 1 through 3,
**characterized in that**
the surfaces are disposed concentrically with each other.

6. The method according to one of the afore mentioned claims,
**characterized in that**
the mixture is introduced between the surfaces on a first side and is evacuated between surfaces on a second side with the temperature of the opposing surface areas dropping from the first side toward the second side.

7. The method according to one of the afore mentioned claims,
**characterized in that**
the mixture is led through, or guided with, heat conductive conveyor belts or flexible tubing.

8. The method according to one of the afore mentioned claims,
**characterized in that**
the at least partially solidified product is freeze-dried by removing one surface and by regulating the temperature of the other surface.

9. The method according to one of the afore mentioned claims,
**characterized in that**
the fluid or pasty mixture used is an aqueous solution or suspension.

10. The method according to one of the afore mentioned claims,
**characterized in that**
an active substance or an additive is added to the fluid or pasty mixture.

11. The method according to one of the afore mentioned claims,
**characterized in that**
biologically active materials, living cells and/or cryoprotectors are added to the fluid or pasty mixture.

12. The method according to one of the afore mentioned claims,
**characterized in that**
an active substance, biologically active materials or living cells are added to the freeze-dried structure.

13. The method according to one of the afore mentioned claims,
**characterized in that**
gasses are additionally dissolved in the mixture.

14. The method according to one of the afore mentioned claims,
**characterized in that**,
after cooling is completed, the mixture is reheated to a recrystallization temperature which is maintained for a certain period of time prior to freeze-drying.

15. The method according to one of the afore mentioned claims,
**characterized in that**
foreign materials are introduced into the fluid or pasty mixture in the desired geometry and orientation and are only removed after freeze-drying.

16. A use of the method according to one of the claims 1 through 15 for manufacturing medical, pharmaceutical products or environment technology and biotechnology products.

17. The use of the method according to one of the claims 1 through 15 for manufacturing sponges for cosmetic or medical purposes.

## Revendications

1. Procédé de fabrication d'éponges selon lequel un mélange liquide ou pâteux de substances est amené à se solidifier du moins en partie avant d'être lyophilisé, le mélange étant refroidi entre deux surfaces de température réglable qui sont espacées l'une de l'autre et ont des températures différentes,
**caractérisé en ce que**
l'on procède à un réglage précis de la température de manière à obtenir une structure ordonnée pendant le processus de solidification.

2. Procédé selon la revendication 1,
**caractérisé en ce que**
l'on maintient sensiblement constante pendant le refroidissement la différence de température entre des portions de surface situées en regard.

3. Procédé selon la revendication 1,
**caractérisé en ce que**
l'on maintient constante la température d'une portion de surface jusqu'à ce que l'autre portion de surface ait refroidi suffisamment pour obtenir le gradient désiré.

4. Procédé selon l'une quelconque des revendications précédentes,
**caractérisé en ce que**
les surfaces sont disposées en parallèle.

5. Procédé selon l'une quelconque des revendications 1 à 3,
**caractérisé en ce que**
les surfaces sont disposées de manière concentrique.

6. Procédé selon l'une quelconque des revendications précédentes,
**caractérisé en ce que**
l'on introduit le mélange entre les surfaces d'un premier côté et qu'on l'évacue entre des surfaces d'un deuxième côté, la température des portions de surface situées en regard étant plus basse du deuxième côté que du premier côté.

7. Procédé selon l'une quelconque des revendications précédentes,
**caractérisé en ce que**
l'on fait passer le mélange par des convoyeurs ou par des tuyaux conducteurs de chaleur ou qu'il est guidé avec ceux-ci.

8. Procédé selon l'une quelconque des revendications précédentes,
**caractérisé en ce que**
l'on lyophilise le produit solidifié du moins en partie en enlevant une surface et en régulant la température de l'autre surface.

9. Procédé selon l'une quelconque des revendications précédentes,
**caractérisé en ce que**
l'on utilise comme mélange fluide ou pâteux une solution ou suspension aqueuse.

10. Procédé selon l'une quelconque des revendications précédentes,
**caractérisé en ce que**
l'on incorpore au mélange fluide ou pâteux une substance active ou un additif.

11. Procédé selon l'une quelconque des revendications précédentes,
**caractérisé en ce que**
l'on ajoute au mélange fluide ou pâteux des matériaux biologiquement actifs, des cellules vivantes et/ou des cryoprotecteurs.

12. Procédé selon l'une quelconque des revendications précédentes,
**caractérisé en ce que**
l'on ajoute à la structure lyophilisée une substance active, des matériaux biologiquement actifs ou des cellules vivantes.

13. Procédé selon l'une quelconque des revendications précédentes,
**caractérisé en ce que**
l'on dissout en outre des gaz dans le mélange.

14. Procédé selon l'une quelconque des revendications précédentes,
**caractérisé en ce que**,
le refroidissement terminé, on réchauffe le mélange en le portant à une température de recristallisation à laquelle on le maintient pendant un temps déterminé avant de le lyophiliser.

15. Procédé selon l'une quelconque des revendications précédentes,
**caractérisé en ce que**
l'on introduit dans le mélange fluide ou pâteux des matières étrangères de manière à obtenir la géométrie et l'orientation souhaitées, ces matières n'étant éliminées qu'après la lyophilisation.

16. Application du procédé selon l'une quelconque des revendications 1 à 15 à la fabrication de produits médicaux, pharmaceutiques ou de produits relevant de la technologie de l'environnement et de la biotechnologie.

17. Application du procédé selon l'une quelconque des revendications 1 à 15 à la fabrication d'éponges destinées à être utilisées à des fins cosmétiques ou médicales.
